# EUROPÄISCHE PATENTSCHRIFT

(11) **EP 1 454 643 B1**
(45) Veröffentlichungstag und Bekanntmachung des Hinweises auf die Patenterteilung: **26.03.2008**
(21) Anmeldenummer: 04010023.2
(22) Anmeldetag: 18.06.2001
(51) Int. Cl.: A61M 1/16

(54) **Verfahren zur Bereitstellung von Dialyseflüssigkeit**
Method of providing dialysis liquid
Procédé pour fournir du liquide de dialyse

(30) Priorität: 17.06.2000 DE 10029892; 14.07.2000 DE 10034368
(43) Veröffentlichungstag der Anmeldung: 08.09.2004
(62) Teilanmeldung aus: 01114555.4
(73) Patentinhaber: Fresenius Medical Care Deutschland GmbH, 61352 Bad Homburg v.d.H. (DE)
(72) Erfinder: Balschat, Klaus, 97525 Schwebheim (DE); Koch, Michael, 97421 Schweinfurth (DE); Wolter, Elmar, 97230 Estenfeld (DE)
(74) Vertreter: Luderschmidt, Schüler & Partner

(56) Entgegenhaltungen:
- DE-C- 19 708 391
- DE-C1- 3 234 119
- US-A- 4 136 708

## Beschreibung

Die Erfindung betrifft ein Verfahren zur Bereitstellung von Dialysierflüssigkeit für eine Dialysevorrichtung.

Zur Herstellung von Dialysierflüssigkeit ist es heute weitgehend üblich, vorgefertigte Dialysekonzentrate einzusetzen, die nur noch mit der entsprechenden Wassermenge verdünnt werden müssen. Um während der Dialyse einen Wärmeentzug aus dem Blut zu vermeiden, wird die Dialysierflüssigkeit auf Körpertemperatur erwärmt. Ferner wird in der Dialysierflüssigkeit gelöste Luft durch Entgasung entfernt.

Der Vorgang des Mischens von Wasser und Konzentrat in einem bestimmten Mengenverhältnis wird im allgemeinen als Proportionierung bezeichnet. Es sind Proportionierungseinrichtungen bekannt, bei denen die Dosierung nicht in einem vorgegebenen Volumenverhältnis, sondern danach erfolgt, daß die entstehende Mischung eine bestimmte elektrische Leitfähigkeit erreicht. Zum Fördern von Wasser und Konzentrat verfügen die leitfähigkeitsgesteuerten Proportionierungseinrichtungen über Pumpen, deren Flußraten in Abhängigkeit von der gemessenen Leitfähigkeit derart geregelt werden, daß sich eine Dialysierflüssigkeit mit der gewünschten Zusammensetzung ergibt.

Wegen der großen Austauschmengen besteht bei den bekannten Dialysevorrichtungen die Notwendigkeit der exakten Bilanzierung von entzogener Flüssigkeit einerseits und zugeführter Flüssigkeit andererseits über die gesamte Behandlungszeit. Zum Stand der Technik gehören volumetrische Bilanziersysteme.

Aus der DE-A-28 38 414 ist eine Dialysevorrichtung bekannt, die ein volumetrisches Bilanziersystem aufweist. Das Bilanziersystem besteht aus zwei durch jeweils eine bewegliche Trennwand unterteilten Kammern, die jeweils eine Zuführleitung für frische und eine Abführleitung für verbrauchte Dialysierflüssigkeit aufweisen. In den Zuführ- und Abführleitungen sind Absperrventile angeordnet, die von einer Steuereinheit angesteuert werden. Das Bilanziersystem wird derart betrieben, dass frische Dialysierflüssigkeit von einer Dialysierflüssigkeitsquelle den beiden Bilanzkammern abwechselnd zugeführt und gleichzeitig verbrauchte Dialysierflüssigkeit abgeführt wird.

Zur Herstellung der Dialysierflüssigkeit wird bei den bekannten Dialysevorrichtungen im allgemeinen Wasser mit einem oder mehreren Konzentraten in einem Reservoir gemischt. Die Konzentratzugabe erfolgt in Abhängigkeit von dem Takt der Bilanzkammer des Bilanziersystems, während die Wasserzugabe über einen im Reservoir befindlichen Füllstandsensor geregelt wird, der in Abhängigkeit des Füllstandsniveaus des Reservoirs das Wassereinlaßventil steuert. Kommt es zu einer Flußratenänderung, so verliert die Proportionierung an Exaktheit. Flußratenänderung treten beispielsweise bei Füllprogrammen auf, wenn bei der Entgasung der vom Dialysator kommenden Flüssigkeit sehr viel Gas anfällt oder wenn sich der hydraulische Widerstand in den Schlauchleitungen ändert. Diese Faktoren bewirken eine Änderung der Taktung der Konzentratpumpen, wobei jedoch die Wasserzufuhr davon nahezu unbeeinflußt bleibt. Diese Gegebenheiten können zu einer Veränderung des Wasser-Konzentrat-Mischungsverhältnisses führen. Zusätzlich haben diese Druck- und Flußänderungen Einfluß auf die Einspritzpunkte der Konzentrate, was ebenfalls zu den Problemen bei der Proportionierung führen kann.

Die DE 30 06 718 beschreibt eine Dialysiervorrichtung, in der die Proportionierung der Dialysierflüssigkeit mittels der Bilanzkammer des Bilanziersystems erfolgt. Hierzu wird Konzentrat bei der Befüllung der Kammer mit Frischwasser zudosiert.

Die GB 2 069 855 A beschreibt eine Dialysevorrichtung mit einer Proportionierungseinrichtung zur Bereitstellung von frischer Dialysierflüssigkeit. Die Proportionierungseinrichtung weist zwei Proportionierungseinheiten A und B auf, die jeweils eine erste und zweite Kammerhälfte aufweisen. Die beiden Kammerhälften jeder Proportionierungseinheit werden wechselweise mit frischer Dialysierflüssigkeit, die dem Dialysator zufließt, und verbrauchter Dialysierflüssigkeit gefüllt, die von dem Dialysator abfließt.

Die DE-C-3234119 beschreibt eine Dialysevorrichtung mit einer Mischeinrichtung zur Erzeugung von Dialysierflüssigkeit aus Konzentrat und Leitungswasser. Frische Dialysierflüssigkeit wird in einem Sammelbehälter gesammelt. Zur Herstellung der frischen Dialysierflüssigkeit wird Leitungswasser, das aus einer Leitung zufließt und Konzentrat, das aus einem Behälter zufließt, in dem Sammelbehälter gemischt. Das Konzentrat wird mittels einer Pumpe gefördert, während das Leitungswasser mit einer Pumpeinrichtung gefördert wird. Die Konzentratpumpe und die Pumpeinrichtung arbeiten taktweise, wobei die Anzahl der Hübe der Konzentratpumpe bzw. Pumpeinrichtung das Mischungsverhältnis von Konzentrat und Leitungswasser bestimmen. Die Pumpeinrichtung verfügt über zwei Kammerhälften, die wechselweise mit Frischwasser befüllt werden. Nachteilig ist beider bekannten Portionierungseinrichtung, dass die Veränderung der Flussrate der Dialysierflüssigkeit einen Einfluss auf die Flussrate der aus dem Sammelbehälter abgeführten Flüssigkeit hat, so dass die Dosierung des Konzentrats nicht exakt vorgenommen werden kann.

Die US-A-4 136 708 beschreibt eine Dialysevorrichtung mit einer Proportionierungseinrichtung, die über einen Sammelbehälter verfügt. Die Konzentrate werden mittels volumetrischer Pumpen exakt dosiert. Konzentrate und Wasser werden in der einen Kammerhälfte einer weiteren volumetrischen Pumpe vorgemischt und dann erst dem Sammelbehälter zugeführt.

Der Erfindung liegt die Aufgabe zugrunde ein Verfahren anzugeben, mit dem Dialysierflüssigkeit für eine Dialysevorrichtung mit hoher Genauigkeit unabhängig von Flussänderungen proportioniert werden kann.

Die Lösung dieser Aufgabe erfolgt erfindungsgemäß mit den Merkmalen des Patentanspruchs 1.

Die Erfindung macht eine Entkopplung von der Konzentratzugabe und der Bilanzierung von zu- und abgeführter Dialysierflüssigkeit möglich. Das erfindungsgemäße Verfahren kann ohne größere Veränderungen des apparativen Aufbaus bei den bekannten Dialysevorrichtungen eingesetzt werden.

Die Vorteile der Erfindung liegen darin, dass die Zugabe von Konzentrat(en) immer bei einer bestimmten Flussrate unabhängig davon erfolgt, welche Dialysierflüssigkeitsrate vorgegeben ist. Da die Mischung von Konzentrat(en) und Wasser immer bei einer bestimmten Flussrate erfolgt, lassen sich Konzentrat und Wasser zur Einstellung einer bestimmten Leitfähigkeit exakt proportionieren.

Die Vorgabe der Flussrate erfolgt dadurch, dass wechselweise die erste und zweite Kammerhälfte einer Proportionierungseinheit befüllt werden, wobei beim Befüllen der einen Kammerhälfte mit Flüssigkeit die Flüssigkeit aus der anderen Kammerhälfte verdrängt wird. Die Flussrate ergibt sich aus dem Volumen der Kammerhälften und der Füll- bzw. Leerzeit, die durch die Umschaltung von in den Zuführ- und Abführleitungen der Kammerhälften angeordneten Absperrorganen exakt vorgegeben werden kann.

Die Proportionierungseinheit kann nur eine Proportionierungskammer umfassen, die durch eine bewegliche Trennwand in zwei Kammerhälften unterteilt ist. Jede Kammerhälfte kann aber auch Teil einer eigenen Proportionierungskammer sein, die jeweils ein separates Verdrängungsorgan aufweisen, die derart miteinander gekoppelt sind, dass beim Befüllen der einen Kammerhälfte Flüssigkeit aus der anderen Kammerhälfte verdrängt wird.

Da bereits fertige Dialysierflüssigkeit bereitgestellt wird, vereinfacht sich der Aufbau des eigentlichen Bilanziersystems insofern, als die Mischung von Wasser und Konzentrat(en) mittels der Bilanzierkammern des Bilanziersystems nicht erforderlich ist.

Die Dialysierflüssigkeit kann aus einem oder mehreren Konzentraten und Wasser gemischt werden, wobei die Mischung von Wasser und Konzentrat(en) an einem oder mehreren Mischpunkten erfolgt. Die Mischpunkte können stromauf oder stromab der Proportionierungseinheit angeordnet sein. Die Zusammensetzung der Mischung aus Wasser und Konzentrat(en) kann zur Überwachung derselben stromab des jeweiligen Mischungspunktes gemessen werden. Dies kann durch eine Leitfähigkeitsmessung oder auch durch eine Dichtemessung erfolgen.

Bei der Proportionierung fließt das Wasser, sofern ein Konzentrat noch nicht zudosiert worden ist oder die Mischung aus Wasser und Konzentrat(en) unabhängig von sonstigen Einflüssen immer mit einer vorgegebenen Flussrate. Dialysatflußabhängig sind also allein die Pausenzeiten zwischen dem Umschalten der Kammerhälften der Proportionierungseinheit.

Um sicherzustellen, dass ein ausreichendes Volumen an Dialysierflüssigkeit bereitgestellt werden kann, wird die fertige Dialysierflüssigkeit gesammelt. Dadurch ist es möglich, in aufeinanderfolgenden Takten zu proportionieren.

Zur Bereitstellung eines bestimmten Reservoirs an frischer Dialysierflüssigkeit können ein Füllstandsgeber und eine Steuereinheit vorgesehen sein, die nach dem Ansinken des Flüssigkeitsspiegels unter einen vorgegebenen Sollwert die Proportionierungseinheit umschaltet, so dass ein bestimmtes Volumen an Flüssigkeit mit einer vorgegebenen Flussrate taktweise gefördert wird.

Vorzugsweise wird die fertige Dialysierflüssigkeit in einer Ausgleichskammer gesammelt, die einen Einlaß zum Zuführen der fertigen Dialysierflüssigkeit und einen Auslaß zum Abführen derselben aufweist, wobei die Dialysierflüssigkeit vorzugsweise mittels einer Pumpe, die in die Versorgungsleitung geschaltet ist, in das Bilanziersystem gefördert wird. Da das Bilanziersystem nicht kontinuierlich , sondern nur taktweise Flüssigkeit fördert, zweigt von der Versorgungsleitung eine Rezirkulationsleitung ab, in die vorzugsweise ein Überdruckventil geschaltet ist. Wenn sich in der Versorgungsleitung ein bestimmter Überdruck aufbaut, kann die überschüssige Dialysierflüssigkeit rezirkulieren.

Vorzugsweise wird mit der Dialysierflüssigkeit mitgeführtes Gas (gelöst oder ungelöst) automatisch abgeschieden. Damit können weitere Luftabscheider grundsätzlich entfallen.

Das Konzentrat oder die einzelnen Konzentrate können in Behältern bereitgestellt werden, beispielsweise Kanister oder Beutel, an denen zu den einzelnen Mischpunkten führende Konzentratleitungen angeschlossen sind. Die Bereitstellung der Konzentrate ist aber auch über eine zentrale Konzentratversorgung möglich.

Das Wasser für die Herstellung der Dialysierflüssigkeit wird vorzugsweise vor dem Eintritt in die Bilanzkammer entgast und/oder erwärmt. Die Entgasungs- und Heizeinheit bleibt frei von Konzentraten, ausgenommen bei Spülvorgängen oder beim Entleeren der Konzentratbeutel.

Im folgenden wird das erfindungsgemäße Verfahren anhand eines Ausführungsbeispiels einer Dialysevorrichtung unter Bezugnahme auf die Zeichnungen näher erläutert.

Es zeigen:
- Figur 1: eine vereinfachte schematische Darstellung einer bevorzugten Ausführungsform einer Dialysevorrichtung mit einer Proportionierungseinrichtung, die nach dem erfindungsgemäßen Verfahren arbeitet,
- Figur 2: ein erstes Ausführungsbeispiel der Ausgleichskammer der Proportionierungseinrichtung von Figur 1 und
- Figur 3: ein zweites Ausführungsbeispiel der Ausgleichskammer der Proportionierungseinrichtung von Figur 1.

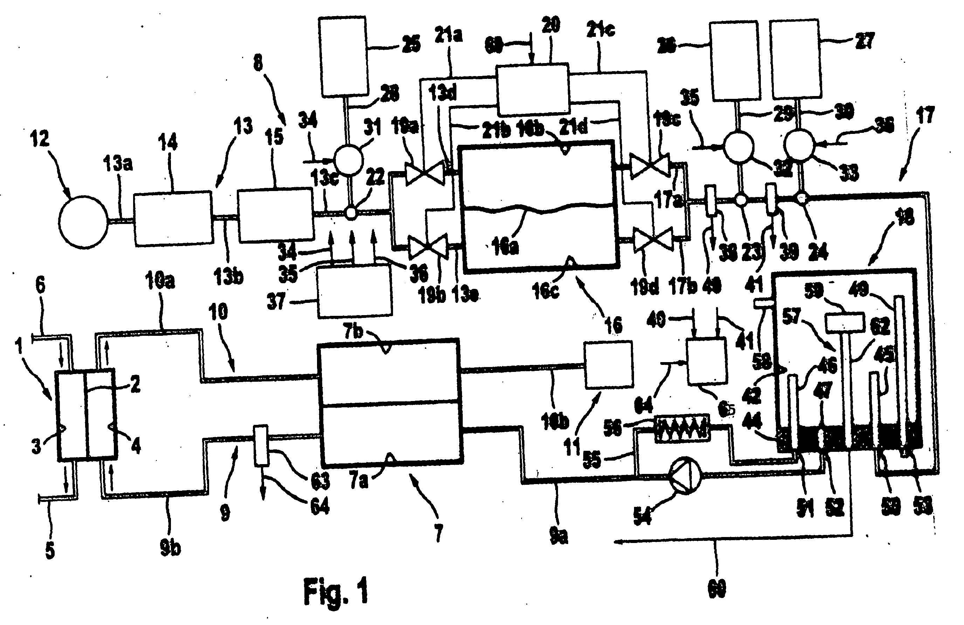

Figur 1 zeigt die wesentlichen Baugruppen einer Dialysevorrichtung in vereinfachter schematischer Darstellung zusammen mit der Proportionierungseinrichtung . Die Dialysevorrichtung weist einen Dialysator 1 auf, der durch einen semipermeable Membran 2 in eine Kammer 3 für eine zu reinigenden Flüssigkeit und einen Dialysierflüssigkeitskammer 4 unterteilt ist. Die zu reinigende Flüssigkeit kann z.B. Blut, weshalb die Kammer nachfolgend als Blutkammer bezeichnet wird, oder beispielsweise aus dem Peritonealraum eines Patienten auslaufenden Flüssigkeit sein. An dem Einlaß der Blutkammer ist eine Blutzuführleitung 6 und an dem Auslaß der Blutkammer eine Blutabführleitung 5 angeschlossen. Zur Bilanzierung von frischer und verbrauchter Dialysierflüssigkeit verfügt die Dialysevorrichtung über ein Bilanziersystem 7, das eine in zwei Bilanzkammerhälften 7a, 7b unterteilte Bilanzkammer aufweist. Das Bilanziersystem kann aber auch über zwei parallel geschaltete Bilanzkammern verfügen.

Die frische Dialysierflüssigkeit wird in einer Proportionierungseinrichtung 8 bereitgestellt, die noch im einzelnen beschrieben wird. Ein erster Abschnitt 9a einer Versorgungsleitung 9 für Dialysierflüssigkeit führt zu einen Einlaß der ersten Kammerhälfte 7a der Bilanzkammer 7, während von einem Auslaß der ersten Bilanzkammerhälfte ein zweiter Abschnitt 9b der Versorgungsleitung abgeht und zu einem Einlaß der Dialysierflüssigkeitskammer 4 führt. Von einem Auslaß der Dialysierflüssigkeitskammer geht ein erster Leistungsabschnitt 10a einer Leitung 10 für verbrauchte Dialysierflüssigkeit ab, die zu einem Einlaß der zweiten Bilanzkammerhälfte 7b der Bilanzkammer führt. Von einem Auslaß der zweiten Bilanzkammerhälfte 7b geht ein zweiter Abschnitt 10b der Leitung 10 ab, der zu einem Abfluß 11 führt. Während des Betriebs der Dialysevorrichtung strömt frische Dialysierflüssigkeit durch die Dialysierflüssigkeitskammer 4 des Dialysators, während im Gegenstrom Blut durch die Blutkammer 3 des Dialysators strömt. Blut- und Dialysierflüssigkeitspumpe in der Blut- und Dialysierflüssigkeitsleitung sind der besseren Übersicht halber nicht dargestellt.

Nachfolgend wird die Proportionierungseinrichtung 8 zur Bereitstellung der Dialysierflüssigkeit im einzelnen beschrieben. Die Proportionierungseinrichtung 8 verfügt über eine Quelle 12 für Frischwasser. Das Frischwasser kann in einem Behälter, beispielsweise einem Tank bereitgestellt werden. Die Proportionierungseinrichtung kann aber auch über eine externe Versorgungsanlage mit ausreichend Frischwasser versorgt werden.

Von der Wasserquelle 12 geht ein erster Abschnitt 13a einer Zuführungsleitung 13 ab, der zu einer Heizeinheit 14 führt, in der das Frischwasser auf Körpertemperatur das Patienten erwärmt wird. Ein zweiter Abschnitt 13b der Zuführung 13 verbindet die Heizeinheit 14 mit einer Entgasungseinheit 15 zum Befreien des Frischwassers von Gas. Von der Entgasungseinheit 15 geht ein dritter Abschnitt 13c der Zuführleitung 13 ab, die zu einer Proportionierungseinheit führt. Die Proportionierungseinheit umfasst vorzugsweise eine Proportionierungskammer, die durch eine bewegliche Trennwand 16a in zwei Proportionierungskammerhälften 16b, 16c unterteilt ist. Die Proportionierungskammer 16 der Proportionierungseinrichtung 8 hat prinzipiell den gleichen Aufbau wie die Bilanzkammer des Bilanziersystems 7 der Dialysevorrichtung. Anstelle einer Proportionierungskammer können aber auch zwei im Gegentakt arbeitende Proportionierungskammern vorgesehen sein. Eine derartige Anordnung ist beispielsweise in der DE-A-197 08 391 beschrieben.

An den Einlaß der ersten Kammerhälfte 16b ist der erste Zweig 13d und an dem Einlaß der zweitem Kammerhälfte 16c der Proportionierungskammer 16 ist der zweite Zweig 13e der Zuführleitung 13 angeschlossen. Eine Abführleitung 17 verbindet die Proportionierungskammer 16 mit einer Ausgleichskammer 18. Die Abführleitung 17 weist einen ersten und zweiten Zweig 17a, 17b auf, wobei der erste Zweig 17a von der ersten Proportionierungskammerhälfte 16c der Proportionierungskammer 16 abgeht. In die Zweige der Zufuhr- und Abführleitung 13, 17 sind jeweils ein Absperrorgan 19a, 19b, 19c, 19d geschaltet. Die Absperrorgane sind elektromagnetische Ventile, die von einer Steuereinrichtung 20 über Steuerleitungen 21a, 21b, 21c, 21d angesteuert werden. Die Steuereinheit 20 steuert die Sperrorgane derart an, dass eine der beiden Kammerhälften der Proportionierungskammer 16 mit Flüssigkeit befüllt wird, wodurch Flüssigkeit aus der jeweils anderen Kammerhälfte verworfen wird. Aus der durch den Ladefluß der Frischwasserquelle 12 und dem Kammervolumen vorgegebenen Füllzeit der Proportionierungskammer ergibt sich eine feststehende Flussrate in der Abführleitung 17. Die Proportionierungskammer 16 in Verbindung mit einer Ausgleichskammer 18 erlaubt es also, Flüssigkeit taktweise mit einer konstanten Flussrate zu fördern, die unabhängig von den Betriebszuständen der anderen Baugruppen der Dialysevorrichtung ist. Die Ausgleichskammer wird nachfolgend noch im einzelnen beschrieben.

Zur Herstellung der fertigen Dialysierflüssigkeit wird das Frischwasser mit drei Konzentraten an drei aufeinanderfolgenden Mischprodukten 22, 23, 24 gemischt. Der erste Mischpunkt 22 befindet sich stromauf der Proportionierungskammer 16 im dritten Abschnitt 13c der Zuführleitung, und der zweite und dritte Mischpunkt 23, 24 befinden sich stromab der Proportionierungskammer 16 in der Abführleitung 17. An den Mischpunkten 22, 23, 24 können in der Zuführ- und Abführleitung 13, 17 beispielsweise Dosierstücke vorgesehen sein. Die drei Konzentrate werden in Behältern, insbesondere Beuteln 25, 26, 27 bereitgestellt, die über Konzentratleitungen 28, 29, 30 sind jeweils Proportionierungspumpen 31, 32, 33 geschaltet, die über Steuerleitungen 34, 35, 36 mit einer zweiten Steuereinheit 37 verbunden sind. Die Proportionierung erfolgt volumetrisch in Abhängigkeit vom Kammervolumen, so dass das gewünschte Mischungsverhältnis erreicht wird. Zum Messen der Leitfähigkeit der Dialysierflüssigkeit stromab des ersten und stromauf des zweiten Mischpunktes 22, 23 ist ein erster Leitfähigkeitssensor 38 vorgesehen, während ein zweiter Leitfähigkeitssensor 39 zum Messen der Leitfähigkeit stromab des zweiten Mischpunktes 23 in der Abführleitung 17 vorgesehen ist. Ein dritter Leitfähigkeitssensor 63 zum Messen der Gesamtleitfähigkeit ist stromauf des Dialysators vorgesehen. Die Messwerte der Leitfähigkeitssensoren 38, 39, 63 werden über Datenleitungen 40, 41, 64 an eine Überwachungseinheit 65 übertragen. Die Steuereinheit 37 gibt die Pump-Volumina der Proportionierungspumpen 31, 32, 33 derart vor, dass sich die Dialysierflüssigkeit mit der gewünschten Zusammensetzung ergibt. Bei der Leitfähigkeitsmessung erfolgt vorzugsweise eine Temperaturkompensation. Die Abführleitung 17 der Proportionierungseinrichtung führt zu der Ausgleichskammer 18.

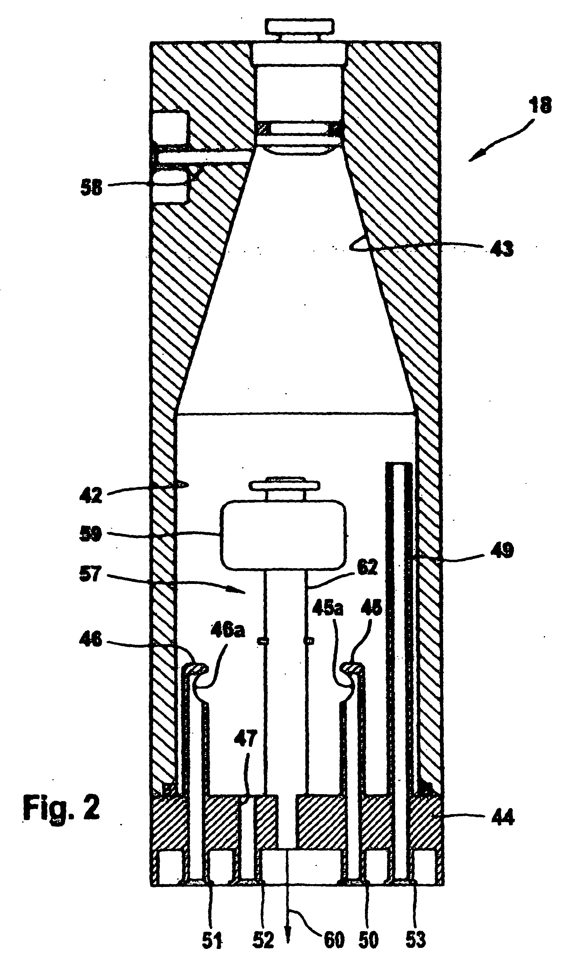

In Figur 1 ist die Ausgleichskammer 18 nur schematisch dargestellt. Die Ausgleichskammer kann unterschiedlich ausgebildet sein. Ein bevorzugtes Ausführungsbeispiel der Ausgleichskammer zeigt Figur 2, die einen im wesentlichen zylindrischen unteren Raum 42 aufweist, an den sich ein im wesentlichen kegelförmiger oberer Raum 43 anschließt. Als Ausgleichskammer kann aber auch die in der deutschen Patentanmeldung 199 29 327.9 beschriebene Kammer verwendet werden.

Figur 1 zeigt der besseren Übersichtlichkeit halber nur die wesentlichen Komponenten der Dialysevorrichtung mit der Proportionierungseinrichtung. Es können auch weitere Komponenten, beispielsweise zusätzliche Sicherheitsventile, Drucksensoren etc. vorgesehen sein.

Alle Anschlüsse der Ausgleichskammer 18 mit Ausnahme des Spülanschlusses sind am Kammerboden 44 vorgesehen. Die Ausgleichskammer weist einen Zulaufstutzen 45 und einen Rezirkulationsstutzen 46 auf, die sich durch den Kammerboden 44 in den Zylinderraum 42 erstrecken. Der Zulauf- und Rezirkulationsstutzen 45, 46 haben jeweils einen senkrecht zur Kammerlängsachse abgewinkelten Flüssigkeitsaustritt 45a, 46a, so dass Flüssigkeit in horizontaler Richtung in den Zylinderraum der Kammer einströmt. Ein Ablaufstutzen 47 ist im Kammerboden 44 vorgesehen. Darüber hinaus erstreckt sich ein Entlüftungsrohr 49 durch den Kammerboden 44 bis nahe an den kegelförmigen Raum 43 der Ausgleichskammer 18, das auch als Überlaufrohr dienen kann.

Der Zulauf-, Abfluß- und Rezirkulationsstutzen sowie das Entlüftungsrohr 49 weisen am Kammerboden Konnektoren 50, 51, 52 uns 53 auf. An dem Konnektor 50 des Zulaufstutzens 45 ist die Abführleitung 17 angeschlossen. Die Versorgungsleitung 9 ist an dem Konnektor 52 des Ablaufstutzens 47 angeschlossen.

In den ersten Abschnitt 9a der Versorgungsleitung 9 ist eine Ladepumpe 54 geschaltet, die fertige Dialysierflüssigkeit aus der Ausgleichskammer abzieht. Stromab der Ladepumpe 54 zweigt von dem ersten Abschnitt 9a der Versorgungsleitung 9 eine Rezirkulationsleitung 55 ab, die an dem Konnektor 51 des Rezirkulationsstutzens 46 angeschlossen ist. In die Rezirkulationsleitung 55 ist ein Überdruckventil 56 geschaltet, das die Strömungsverbindung in die Ausgleichskammer nur bei Überschreiten eines bestimmten Überdrucks freigibt.

Zur Überwachung des Füllstandes der Ausgleichskammer ist ein Füllstandgeber 57 vorgesehen. Der Füllstandgeber 57 weist einen im Zentrum des Zylinderraums 42 angeordneten Stab 62 auf, an dem ein Schwimmer 59 im Kammerlängsrichtung verschiebbar geführt ist. Wenn der Flüssigkeitsspiegel in der Ausgleichskammer einen vorgegebenen Sollwert unterschreitet, gibt der Füllstandgeber über ein Datenleitung 60 ein Steuersignal an die Steuereinheit 20 ab.

Zum Abschneiden von Luft, die in der fertigen Dialysierflüssigkeit mitgeführt werden kann, ist ein Entlüftungsrohr vorgesehen. Zu Spülen der Ausgleichskammer ist ein Spülrohr 58 vorgesehen, das an dem oberen Ende des kegelförmigen Raums 43 in die Ausgleichskammer 18 mündet. An dem Spülrohr 58 kann eine nicht dargestellte Spülleitung angeschlossen werden, über die Spülflüssigkeit in die Ausgleichskammer strömt. Die Spülflüssigkeit fließt dann an der Innenwand des kegelförmigen Raumes 43 und des Zylinderraumes 42 nach unten, so dass die gesamte Kammer vollständig benetzt wird.

Nachfolgend wird die Funktion der Proportionierungseinrichtung im einzelnen beschrieben. Die Steuereinheit 20 steuert die Absperrorgane derart an, dass die Absperrorgane 19a und 19d geöffnet und die Absperrorgane 19b und 19c geschlossen sind. Das in der Heizeinheit 14 erwärmte und in der Entgasungseinheit 15 entgaste Frischwasser aus der Wasserquelle 12 strömt über den ersten Zweig 13d der Zuführleitung 13 in die erste Kammerhälfte 16d der Proportionierungskammer 16. Während des Befüllens der ersten Kammerhälfte 16d wird ein speziell für den Patienten vorgesehenes Individualkonzentrat aus dem Beutel 25 mittels der Proportionierungspumpe 31, deren Flussrate von der Steuereinheit 20 vorgegeben wird, an dem ersten Mischpunkt 22 zudosiert. Die Füllzeit der ersten Kammerhälfte beträgt vorzugsweise 1,2 Sekunden, was einer Flussrate in der Abführleitung 17 von 150 ml/min entspricht. Der Dosiervorgang beginnt mit dem Öffnen der Absperrorgane 19a, 19d bzw. nach dem Schließen der Absperrorgane 19b, 19c und endet nach ca. 0,9-1,0 Sekunden, so dass ausreichend Zeit verbleibt, um die gesamte Menge an Individualkonzentrat in die erste Kammerhälfte zu befördern.

Beim Befüllen der ersten Kammerhälfte 16b drückt die bewegliche Membran die Mischung aus Frischwasser und Individualkonzentrat der zweiten Kammerhälfte aus einem vorhergehenden Takt in die Abführleitung 17. An dem zweiten Mischpunkt 23 wird Bicarbonatkonzentrat aus dem Beutel 26 mittels der Proportionierungspumpe 32 zudosiert und an dem dritten Mischpunkt 24 wird Säurekonzentrat aus dem Beutel 27 mittels der Proportionierungspumpe 33 zudosiert. Die Leitfähigkeit der Mischung aus Frischwasser und Individualkonzentrat wird mittels des ersten Leitfähigkeitssensor 38 überwacht. Die Leitfähigkeit der Bicarbonatdosierung wird mittels des zweiten Leitfähigkeitssensor 39 überwacht, wobei durch Verrechnung der Messwerte auf die Bicarbonatdosierung geschlossen werden kann. Die Gesamtleitfähigkeit der Dialysierflüssigkeit wird mittels des dritten Leitfähigkeitssensor 63 stromab der Ausgleichskammer 18 und stromauf des Dialysators 1 überwacht, der über eine Datenleitung 64 mit der Überwachungseinheit 65 verbunden ist. Die Dosierung von Bicarbonat- und Säurekonzentrat beginnt mir dem Öffnen der Absperrorgane 19a, 19d bzw. nach dem Schließen der Absperrorgane 19b, 19cc. Die Dosierzeit von Bicarbonat und Säure ist so bemessen, dass die gesamte Menge an Konzentraten durch die Abführleitung 17 in die Ausgleichskammer 18 gefördert wird. Die Flussrate, mit der die Flüssigkeit durch die Abführleitung strömt, ist allein abhängig vom Ladefluß der Frischwasserquelle und dem Kammervolumen. Wenn die Flussrate der Dialysierflüssigkeit durch die Dialysierflüssigkeitskammer 4 des Dialysators verändert werden sollte, hat dies keinen Einfluß auf die Flussrate in der Abführleitung. Dies hat den Vorteil, dass die Leitfähigkeit mit hoher Genauigkeit unabhängig von Flussveränderungen gemessen und die Dosierung der Konzentrate exakt vorgenommen werden kann.

Anstelle der Individualkonzentratdialyse kann aber auch eine Acetatdialyse, bei der Acetat nur an dem dritten Mischpunkt 24 zudosiert wird, oder eine Bicarbonatdialyse durchgeführt werden, bei der an dem zweiten Mischpunkt 23 Bicarbonat und an dem dritten Mischpunkt 24 Säurekonzentrat, aber kein Individualkonzentrat zugeführt wird.

Die Ausgleichskammer 18 stellt ein variables Puffervolumen zwischen der Proportionierungskammer 16 der Proportionierungseinrichtung und dem Bilanziersystem 7 der Dialysevorrichtung dar. Sie gleicht Volumentoleranzen aus und fördert die Durchmischung der Dialysierflüssigkeit. Des weiteren dient sie der Entlüftung.

Die Ausgleichskammer 8 wird taktweise mit Dialysierflüssigkeit befüllt, wobei wechselweise die erste bzw. zweite Kammerhälfte 16b, 16c der Proportionierungskammer 16 unter Verwerfung der Flüssigkeit aus der jeweils anderen Kammerhälfte befüllt werden. Um die zweite Kammerhälfte 16c zu befüllen, steuert die Steuereinheit 20 die Absperrorgane derart an, dass die Absperrorgane 19b und 19c geöffnet und die Absperrorgane 19a und 19d geschlossen sind.

Die fertige Dialysierflüssigkeit wird aus der Ausgleichskammer 18 mittels der Ladepumpe 54 abgezogen und der Bilanzkammer des Bilanziersystems 7 der Dialysevorrichtung zugeführt. Die Ladezeit der Bilanzkammer des Bilanziersystems der Dialysevorrichtung ist vorzugsweise 1,5 Sekunden, was einer Flussrate von 1.200 ml/min entspricht. Nach Ende der Bilanzkammerfüllzeit steigt der Ladedruck, so dass das Überdruckventil 56 in der Rezirkulationsleitung 55 öffnet und die Dialysierflüssigkeit wieder in die Ausgleichskammer zurückgeführt wird.

Die Ansteuerung der Absperrorgane der Proportionierungskammer 16 der Proportionierungseinrichtung 8 erfolgt in Abhängigkeit von dem Steuersignal des Füllstandsgebers 57 in der Ausgleichskammer 18. Sinkt der Flüssigkeitsspiegel in der Ausgleichskammer unter den vorgegebenen Sollwert, dann steuert die Steuereinheit die Absperrorgane derart an, dass die Proportionierungskammer 16 umgeschaltet wird. Die nächste Proportionierungskammerumschaltung kann frühestens 1,5 Sekunden nach der letzten Umschaltung erfolgen. Dadurch ist sichergestellt, dass die Ladezeit der Proportionierungskammer 16 von 1,2 Sekunden nicht unterschritten wird. Steigt nach einer Umschaltung der Proportionierungskammer 16 der Flüssigkeitsspiegel in der Ausgleichskammer nicht soweit an, dass der Schaltpunkt des Füllstandgebers überschritten wird, dann erfolgt nach einer kurzen Totzeit, in der die Steuereinheit 20 alle Absperrorgane schließt, eine weitere Umschaltung der Proportionierungskammer 16. Dies geschieht so oft, bis der gewünschte Flüssigkeitsspiegel in der Ausgleichskammer 18 erreicht ist. Da das Volumen der Proportionierungskammer 16 Proportionierungseinrichtung 8 und das Volumen der zu dosierenden Konzentrate etwa gleich dem Volumen der Bilanzkammer 16 des Bilanziersystems 7 der Dialysevorrichtung ist, schalten beide Kammern in der Regel synchron um.

Nur in einzelnen Fällen erfolgen zwei Umschaltungen der Proportionierungskammer 16 unmittelbar hintereinander. Die Pausenzeiten zwischen zwei Umschaltungen verändern sich in Abhängigkeit von der eingestellten Flussrate der Dialysierflüssigkeit. Beispielsweise beträgt bei einem Dialysierflüssigkeitsfluß von 1.000 ml/min ein Bilanzkammerzyklus 1,8 Sekunden und bei 100 ml/min 18 Sekunden. Für den Fall einer Störung kann überflüssige Dialysierflüssigkeit über das Entlüftungsrohr 49 abfließen.

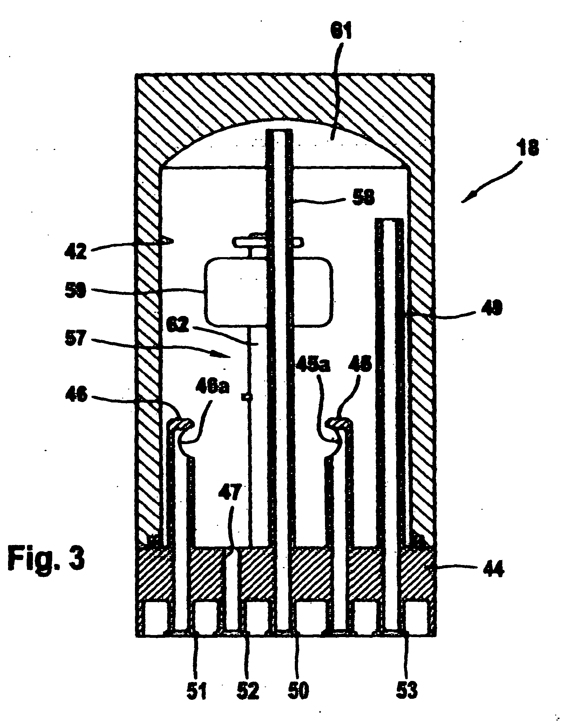

Figur 3 zeigt ein zweites Ausführungsbeispiel der Ausgleichskammer. Die Teile der Ausgleichskammer von Figur 3, die denjenigen der Kammer von Figur 2 entsprechen, sind mit den gleichen Bezugszeichen versehen. Die Kammer von Figur 3 unterscheidet sich von der Kammer von Figur 2 zum einen durch die Gehäuseform und zum anderen durch die Anordnung des Spülrohres.

Die Ausgleichskammer weist nicht einen kegelförmigen, sondern einen nach außen gewölbten Behälterdeckel 61 (Dom) auf. Das Spülrohr 58 mündet nicht seitlich in die Kammer, sondern erstreckt sich durch den Kammerboden 44 nach oben bis kurz vor die Domwandung. Die Reinigung der Ausgleichskammer erfolgt dadurch, dass die Spüllösungen unter Druck von unten auf die Domwandung gesprüht wird. Die Spüllösung läuft dann gleichmäßig an allen Seiten an der Wandung nach unten , so dass sie gesamte Innenfläche der Kammer gereinigt wird. Ansonsten hat die Ausgleichskammer von Figur 3 die gleiche Funktion wie die Kammer von Figur 2.

## Patentansprüche

1. Verfahren zur Bereitstellung von Dialysierflüssigkeit für eine Dialysevorrichtung mit einem Bilanzsystem zum Bilanzieren von frischer und verbrauchter Dialysierflüssigkeit, wobei
wechselweise eine erste und eine zweite Kammerhälfte mindestens einer Proportionienmgseinheit (16) mit Wasser oder einer Mischung aus Wasser und Dialysierflüssigkeitskonzentrat(en) unter Verwerfung der Flüssigkeit aus der jeweils anderen Kammerhälfte befüllt wird, so dass für die Flüssigkeit, mit der die jeweilige Kammerhälfte befüllt oder mit der die Flüssigkeit aus der jeweiligen Kammerhälfte verworfen wird, eine bestimmte Flussrate vorgegeben wird und
der aus der jeweiligen Kammerhälfte mit der vorgegebenen Flussrate verworfenen Flüssigkeit und/oder der jeweiligen Kammerhälfte mit der vorgegebenen Flussrate zugeführten Flüssigkeit ein oder mehrere Dialysierflüssigkeitskonzentrat(e) zur Herstellung der frischen Dialysierflüssigkeit zudosiert werden, bevor die frische Dialysierflüssigkeit gesammelt wird, und dann erst die frische Dialysierflüssigkeit gesammelt wird.

2. Verfahren nach Anspruch 1, **dadurch gekennzeichnet, dass** die frische Dialysierflüssigkeit in einer Ausgleichskammer (18) gesammelt wird, deren Füllstand überwacht wird, und dass nach dem Absinken des Flüssigkeitsspiegels unter einen vorgegebenen Sollwert die Proportionierungseinheit so lange umgeschaltet wird, bis der Flüssigkeitsspiegel wieder über den Sollwert liegt.

3. Verfahren nach Anspruch 2, **dadurch gekennzeichnet, dass** beim Abführen der Dialysierflüssigkeit aus der Ausgleichskammer ein Teil der abgeführten Flüssigkeit wieder in die Ausgleichskammer zurückgeführt wird.

4. Verfahren nach Anspruch 2 oder 3, **dadurch gekennzeichnet, dass** in der Dialysierflüssigkeit enthaltenen Luft in der Ausgleichskammer abgeschieden wird.

5. Verfahren nach einem der Ansprüche 1 bis 4, **dadurch gekennzeichnet, dass** das Wasser oder die Mischung aus Wasser und Dialysierflüssigkeit vor den Eintritt in die Proportionierungseinheit entgast und/oder erwärmt wird.

6. Verfahren nach einem der Ansprüche 1 bis 5, **dadurch gekennzeichnet, dass** zum Messen der Zusammensetzung der Mischung aus Wasser und Dialysierflüssigkeitskonzentrat(en) die Leitfähigkeit der Mischung stromab eines Mischpunktes gemessen wird.

## Claims

1. A method for providing dialysing fluid for a dialysis apparatus with a balancing system for balancing fresh and used dialysing fluid, whereby
a first and a second chamber half of at least one proportioning unit (16) is alternately filled with water or a mixture of water and dialysing fluid concentrate(s), the fluid from the respective other chamber half thereby being discarded, so that a specific flow rate is preset for the fluid with which the respective chamber half is filled and at which the fluid from the respective chamber half is discarded and,
before the fresh dialysing fluid is collected, one or more dialysing fluid concentrate(s) for the preparation of the fresh dialysing fluid are fed in a metered fashion to the fluid discarded at the preset flow rate from the respective chamber half and/or to the fluid fed at the preset flow rate to the respective chamber half, and only then is the fresh dialysing fluid collected.

2. The method according to claim 1, **characterised in that** the fresh dialysing fluid is collected in a compensating chamber (18), the level whereof is monitored, and that, after the liquid level has fallen below a preset setpoint value, the proportioning unit is switched over until such time as the liquid level again lies above the setpoint value.

3. The method according to claim 2, **characterised in that**, when the dialysing fluid is carried away from the compensating chamber, a part of the fluid carried away is fed back again into the compensating chamber.

4. The method according to claim 2 or 3, **characterised in that** air contained in the dialysing fluid is separated in the compensating chamber.

5. The method according to any one of claims 1 to 4, **characterised in that** the water or the mixture of water and dialysing fluid is degassed and/or heated before entry into the proportioning unit.

6. The method according to any one of claims 1 to 5, **characterised in that**, in order to measure the composition of the mixture of water and dialysing fluid concentrate(s), the conductivity of the mixture is measured downstream of a mixing point.

## Revendications

1. Procédé d'approvisionnement d'un liquide de dialyse pour un dispositif de dialyse avec un système de bilan pour établir le bilan du liquide de dialyse frais et usagé, dans lequel
■ une première et une seconde moitié de chambre d'au moins une unité de proportionnement (16) sont remplies alternativement avec de l'eau ou un mélange d'eau et de concentré (s) de liquide de dialyse en fonction du rejet du liquide de chaque autre moitié de chambre, de sorte qu'un certain débit est prédéfini pour le liquide dont est rempli la moitié de chambre respective ou avec lequel est rejeté le liquide de la moitié de chambre respective et
■ un ou plusieurs concentrés de liquide de dialyse sont ajoutés au liquide rejeté de la moitié de chambre respective avec le débit prédéfini et/ou au liquide amené à la moitié de chambre respective avec le débit prédéfini pour obtenir le liquide de dialyse frais, avant que le liquide de dialyse frais soit collecté, et ensuite dès que le liquide de dialyse frais est collecté.

2. Procédé selon la revendication 1, **caractérisé en ce que** le liquide de dialyse frais est collecté dans une chambre d'équilibrage (18), dont le niveau de remplissage est contrôlé/surveillé, et **en ce que** lorsque le niveau du liquide passe en dessous d'une valeur de consigne prédéfinie, l'unité de proportionnement est commutée tant que le niveau du liquide ne dépasse pas à nouveau la valeur de consigne.

3. Procédé selon la revendication 2, **caractérisé en ce que** lors de l'évacuation du liquide de dialyse de la chambre d'équilibrage, une partie du liquide évacué retourne dans la chambre d'équilibrage.

4. Procédé selon la revendication 2 ou 3, **caractérisé en ce que** l'air contenu dans le liquide de dialyse est extrait dans la chambre d'équilibrage.

5. Procédé selon l'une des revendications 1 à 4, **caractérisé en ce que** l'eau ou le mélange d'eau et de liquide de dialyse est évaporé(e) et/ou chauffé(e) avant l'entrée dans l'unité de proportionnement.

6. Procédé selon l'une des revendications 1 à 5, **caractérisé en ce que** pour mesurer la composition du mélange d'eau et de concentré(s) de liquide de dialyse, on mesure la conductivité du mélange en aval d'un point de mélange.
